# EUROPEAN PATENT APPLICATION

(11) **EP 3 438 248 A1**
(43) Date of publication of application: **06.02.2019**
(21) Application number: 17775383.7
(22) Date of filing: 30.03.2017
(51) Int. Cl.: C12N 5/0735, C12N 1/00, C12N 5/10

(54) **NAÏVE PLURIPOTENTIAL STEM CELL CULTURING MEDIUM AND PLURIPOTENTIAL STEM CELL CULTURING METHOD**

(30) Priority: 30.03.2016 JP 2016068718
(71) Applicant: KYOWA HAKKO BIO CO., LTD., Tokyo 100-8185 (JP)
(72) Inventor: FURUE, Miho, Ibaraki-shi Osaka 567-0085 (JP); SHOJI, Shinichiro, Tokyo 1008185 (JP); YANAGIHARA, Kana, Tokyo 100-8185 (JP); TSUKAHARA, Masayoshi, Tokyo 100-8185 (JP)
(74) Representative: Vossius & Partner Patentanwälte Rechtsanwälte mbB
(86) International application number: PCT/JP2017/013205
(87) International publication number: WO 2017/170849

(57) **Abstract**

An object of the present invention is to provide a medium for culturing naive pluripotent stem cells which is for efficiently proliferating naive pluripotent stem cells while maintaining the undifferentiated state and the pluripotency (pluripotent capability) of the naive pluripotent stem cells. The medium of the invention contains a ROCK inhibitor at a concentration of 5 µM or less

## Description

### Technical Field

An object of the present invention is to provide a medium for culturing naive pluripotent stem cells which is for efficiently proliferating naive pluripotent stem cells while maintaining the undifferentiated state and the pluripotency (pluripotent capability) of the naive pluripotent stem cells. Another object of the invention is to provide a method for proliferating pluripotent stem cells while maintaining the undifferentiated state and the pluripotency (pluripotent capability) of the pluripotent stem cells and while maintaining the naive state.

### Background Art

Human iPS cells can be induced by the introduction of three or four factor genes to somatic cells and are cells having the ability to differentiate into almost all the cells in the living body (pluripotency). Due to the characteristics, it is expected that human iPS cells are applied to regenerative medicine for treating diseases which have been difficult to treat so far, by artificially producing a tissue from iPS cells and transplanting the tissue into the body of a patient. Moreover, because it may be possible to produce human cells which have not been available, it is expected that human iPS cells are also applied to models for analyzing diseases and to screening such as evaluation of the effects or the safety of novel drugs.

Human pluripotent stem cells including human iPS cells can be subcultured and maintained by on-feeder culture using fetal fibroblasts of mouse, human or the like, placenta-derived cells or the like as feeder cells or by feeder-free culture using Matrigel, extracellular matrix such as vitronectin, laminin or fibronectin or a flask having a surface in which a recombinant protein or the like is coated with extracellular matrix.

In these methods, however, cells are cultured two-dimensionally in the state of being adhered to the flask surface, and thus these methods are not efficient for large-scale preparation of cells. It is expected that a large number of cells can be cultured at low cost when human iPS cells can be cultured three-dimensionally in suspension culture.

Adherent cells cannot be cultured in suspension culture unless microcarrier beads or the like are used. It is known that human pluripotent stem cells form aggregates and differentiate spontaneously when they are suspended. Also, it is necessary to disperse human iPS cells into single cells or clusters of several to several dozen cells when human iPS cells are cultured in suspension culture, and human iPS cells have a problem of apoptosis caused at this point. With respect to this problem, it is known that the addition of Y-27632, which is a ROCK (Rho-associated coiled-coil forming kinase) inhibitor, to the medium promotes the intercellular adhesion and suppresses the apoptosis of human iPS cells caused when the cells are dispersed into single cells or clusters of several to several dozen cells (NPL 1).

Recently, pluripotent stem cells are classified into "naive type" and "primed type". In the case of mouse, conditions for culturing the naive type having the properties of pre-implantation epiblast and conditions for culturing the primed type having the properties of post-implantation epiblast are known. The "naive type" is in a more undifferentiated state, has greater ability to produce a chimeric individual, especially has the ability to differentiate into germ cells such as sperm or eggs and thus is superior to the primed type in quality. Also, naive cells form multi-layered dome-shaped colonies and proliferate uniformly and stably. There is also a report that naive cells can be cultured in serum-free suspension culture in the form of aggregates (NPL 2). In the case of human, it is known that cells become the "primed type" when the cells are produced from an inner cell mass of a pre-implantation fertilized egg. Human iPS cells are also the primed type.

Y-27632 is generally added at a concentration of 10 µM in the already reported suspension culture systems for human iPS cells (NPLs 3 to 8), and the human iPS cells in these cases are believed to be the "primed type" from the shapes of the cells and the expression patterns of marker genes.

It is reported that mouse ES cells transform into the naive type when the cells are cultured in a 2i/L medium containing a MEK inhibitor, a GSK inhibitor and LIF. On the other hand, it is known that mouse epiblast stem cells and human pluripotent stem cells differentiate when the cells are cultured in a 2i/L medium.

Reported methods for inducing human iPS cells into the "naive type" are methods in which the "naive type" is induced by feeder culture or feeder-free culture using a medium containing any one or more signal inhibitors of a MEK inhibitor (PD0325901), a GSK inhibitor (CHIR99021, IM12 or BIO), a JNK inhibitor (SP600125), a P38 inhibitor (SB203580), a PKC inhibitor (Go6983), a ROCK inhibitor (Y-27632), a BMP inhibitor (Dorsomorphin), a BRAF inhibitor (SB590885) and a SRC inhibitor (WH-4-023) (NPLs 9 to 14).

The points that are common to the methods for inducing the "naive type" are that the medium always contains a MEK inhibitor and a GSK inhibitor and that the cells are proliferated by adhesion culture using feeder cells or Matrigel. It is known that the expression levels of genes peculiar to the "naive type" such as STELLA and TFCP2L1 are increased significantly in the "naive" cells induced by these methods as compared to those in the "primed type" observed in general media.

No example which shows that "naive" human iPS cells are induced by culturing human iPS cells without adding any MEK inhibitor has been known.

### Citation List

### Non-Patent Literatures

NPL 1: Watanabe K. el. Al., NATURE BIOTECHNOLOGY VOLUME 25, p.681
NPL 2: Hayashi Yohei et al. Stem Cells. 25, 3005-15 (2007)
NPL 3: Ayumi Horiguchi et al. Journal of Bioscience and Bioengineering VOL. 118 No. 5, 588-592, 2014
NPL 4: Methods Enzymol., 325 (2000), pp. 273-284
NPL 5: Nature Biotechnology 25, 681 - 686 (2007)
NPL 6: Guokai Chen et al. Cell Stem Cell 7, 240-248 (2010)
NPL 7: Nil Emre et al. PLoS One 5, e12148 (2010)
NPL 8: Yuehong Wu et al. Theriogenology (2015) [Epub ahead of print]
NPL 9: Yasuhiro Takashima et al., Cell 158, 1254-1269 (2014)
NPL 10: Thorold W. Theunissen et al. Cell Stem Cell 15, 471-487 (2014)
NPL 11: Carol B. Ware et al. PNAS 111, 4484-4489 (2014)
NPL 12: Bahram Valamehr et al. Stem Cell Reports 2, 366-381 (2014)
NPL 13: Yun-Shen Chan et al. Cell Stem Cell 13, 663-675 (2013)
NPL 14: Ohad Gafni et al. Nature 504, 282-286 (2013)

### Summary of Invention

### Technical Problem

An object of the present invention is to provide a medium for culturing naive pluripotent stem cells which is for efficiently proliferating naive pluripotent stem cells while maintaining the undifferentiated state and the pluripotency (pluripotent capability) of the naive pluripotent stem cells.

### Solution to Problem

The present inventors have found that the above problems can be solved when a ROCK inhibitor at a low concentration is present in the medium, and the inventors have thus completed the invention.

That is, the present invention relates to the following (1) to (9).
(1) A medium for culturing naive pluripotent stem cells containing a ROCK inhibitor at a concentration of 5 µM or less.
(2) The medium for culturing naive pluripotent stem cells described in (1), wherein the ROCK inhibitor is Y-27632.
(3) The medium for culturing naive pluripotent stem cells described in (1) or (2) which does not contain a MEK inhibitor.
(4) The medium for culturing naive pluripotent stem cells described in any one of (1) to (3) which does not contain feeder cells and extracellular matrix.
(5) The medium for culturing naive pluripotent stem cells described in any one of (1) to (4) which further contains a growth factor.
(6) The medium for culturing naive pluripotent stem cells described in any one of (1) to (5) which is used for suspension culture.
(7) The medium for culturing naive pluripotent stem cells described in any one of (1) to (6), wherein the naive pluripotent stem cells are human iPS cells.
(8) A method for culturing pluripotent stem cells characterized by culturing naive pluripotent stem cells in a medium containing a ROCK inhibitor at a concentration of 5 µM or less and thus proliferating the pluripotent stem cells while maintaining the naive state of the pluripotent stem cells.
(9) The method for culturing pluripotent stem cells described in (8), wherein the ROCK inhibitor is Y-27632.
(10) The method for culturing pluripotent stem cells described in (8) or (9), wherein the medium does not contain a MEK inhibitor.
(11) The method for culturing pluripotent stem cells described in any one of (8) to (10), wherein the medium does not contain feeder cells and extracellular matrix.
(12) The method for culturing pluripotent stem cells described in any one of (8) to (11) which is conducted in suspension culture.
(13) The method for culturing pluripotent stem cells described in any one of (8) to (12), wherein the naive pluripotent stem cells are human iPS cells.

### Effects of the Invention

According to the medium for culturing naive pluripotent stem cells of the invention, because a ROCK inhibitor at a low concentration is contained, naive pluripotent stem cells can be efficiently proliferated while the undifferentiated state and the pluripotency (pluripotent capability) of the naive pluripotent stem cells are maintained. Moreover, according to the method for culturing pluripotent stem cells of the invention, by culturing naive pluripotent stem cells in a medium containing a ROCK inhibitor at a low concentration, the naive state of the pluripotent stem cells can be efficiently maintained while the undifferentiated state and the pluripotency (pluripotent capability) are maintained.

### Brief Description of Drawings

[Fig. 1] Fig. 1A and Fig. 1B are figures showing the results of observation of the proliferation of iPS cells in the presence of a ROCK inhibitor.
[Fig. 2] Fig. 2 is a graph showing the results of measurement of the number of iPS cells and the viability of iPS cells in the presence of a ROCK inhibitor.
[Fig. 3] Fig. 3A to Fig. 3D are graphs which show the results of real-time PCR examining the expression levels, in the presence of a ROCK inhibitor, of NANOG and OCT3/4, which are marker genes for undifferentiated iPS cells, STELLA, which is a naive type-specific marker gene, and NCAM, which is a middle lobe differentiation-specific marker gene, respectively.
[Fig. 4] Fig. 4 is a graph showing the proliferation of iPS cells in the presence of a ROCK inhibitor at a low concentration and growth factors.
[Fig. 5] Fig. 5A to Fig. 5D are graphs showing the expression of marker genes for undifferentiated iPS cells and naive type-specific marker genes in the presence of a ROCK inhibitor at a low concentration and growth factors.
[Fig. 6] Fig. 6 is a graph showing the growth rates of iPS cells in the presence of a ROCK inhibitor at a low concentration and low molecular weight compounds.
[Fig. 7] Fig. 7A and Fig. 7B are graphs showing the expression of marker genes for undifferentiated iPS cells in the presence of a ROCK inhibitor at a low concentration and low molecular weight compounds.

### Embodiments for Carrying Out the Invention

### 1. Medium for Culturing Naive Pluripotent Stem Cells

The invention relates to a medium for culturing naive pluripotent stem cells containing a ROCK inhibitor at a concentration of 5 µM or less (hereinafter also called "the medium of the invention"). The medium of the invention is obtained by adding a ROCK inhibitor at 5 µM or less to a basal medium for pluripotent stem cells.

The basal medium for pluripotent stem cells is not particularly limited as long as the medium can efficiently proliferate naive pluripotent stem cells while maintaining the undifferentiated state and the pluripotency (pluripotent capability) of the naive pluripotent stem cells when a ROCK inhibitor is added at a concentration of 5 µM or less. A chemically synthesized medium is preferable because the preparation is easy and the variation among lots can be prevented.

The basal medium for pluripotent stem cells preferably contains one or more kinds of saccharide(s), one or more kinds of inorganic salt(s), one or more kinds of amino acid(s), one or more kinds of vitamin(s) and one or more kinds of minor component(s). Moreover, the basal medium can also appropriately contain an antibiotic such as kanamycin so that the basal medium can be used for a drug susceptibility test.

Examples of the saccharide(s) include monosaccharides such as glucose, lactose, mannose, fructose and galactose and disaccharides such as sucrose, maltose and lactose. Of these examples, glucose is particularly preferable. One of the saccharides or a combination of two or more thereof can be added.

Examples of the inorganic salt(s) include calcium chloride, calcium nitrate, copper sulfate pentahydrate, iron(III) nitrate nonahydrate, iron(II) sulfate heptahydrate, magnesium chloride hexahydrate, magnesium sulfate, potassium chloride, sodium chloride, disodium hydrogen phosphate, disodium hydrogen phosphate dihydrate, sodium dihydrogen phosphate, sodium dihydrogen phosphate dihydrate and zinc sulfate heptahydrate. Any inorganic salt or a combination of inorganic salts can be used as long as the inorganic salt(s) is a component which advantageously acts on the maintenance of the undifferentiated state of the pluripotent stem cells.

Examples of the amino acid(s) include alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, valine and the like, and L-amino acids are preferable. The amino acid(s) can include products derived from the amino acids such as derivatives, salts and hydrates.

Examples of the products derived from arginine include L-arginine hydrochloride, L-arginine monohydrochloride and the like. Examples of the products derived from aspartic acid include L-aspartic acid sodium salt monohydrate, L-aspartic acid monohydrate, potassium L-aspartate, magnesium L-aspartate and the like.

Examples of the products derived from cysteine include L-cysteine dihydrochloride, L-cysteine hydrochloride monohydrate and the like. Examples of the products derived from lysine include L-lysine hydrochloride and the like. The products derived from glutamic acid include monosodium L-glutamate and the like.

Examples of the products derived from asparagine include L-asparagine monohydrate and the like. Examples of the products derived from tyrosine include L-tyrosine disodium salt dihydrate and the like. Examples of the products derived from histidine include histidine hydrochloride, histidine hydrochloride monohydrate and the like. Examples of the products derived from lysine include L-lysine hydrochloride and the like.

Examples of the vitamin(s) include ascorbic acid, biotin, choline, folic acid, inositol, niacin, pantothenic acid, pyridoxine, riboflavin, thiamine, vitamin B12 and para-aminobenzoic acid (PABA). Of these examples, ascorbic acid is preferably added. The vitamin(s) can include products derived from the vitamins such as derivatives, salts and hydrates.

Examples of the products derived from ascorbic acid include ascorbic acid 2-phosphate, magnesium ascorbyl phosphate, sodium ascorbyl sulfate, aminopropyl ascorbyl phosphate, sodium ascorbyl phosphate and the like.

Examples of the products derived from choline include choline chloride and the like. Examples of the products derived from niacin include nicotinic acid, nicotinamide, nicotinic alcohol and the like. Examples of the products derived from pantothenic acid include calcium pantothenate, sodium pantothenate, panthenol and the like.

Examples of the products derived from pyridoxine include pyridoxine hydrochloride, pyridoxal hydrochloride, pyridoxal phosphate, pyridoxamine and the like. Examples of the products derived from thiamine include thiamine hydrochloride, thiamine nitrate, bisthiamine nitrate, thiamine dicetyl sulfate, fursultiamine hydrochloride, octotiamine, benfotiamine and the like.

The minor component is preferably a component which advantageously acts on the maintenance of the undifferentiated state of pluripotent stem cells. Examples of the minor component include components which are generally used as components of media such as glutathione, hypoxanthine, lipoic acid, linolenic acid, phenol red, putrescine, pyruvic acid, thymidine and NaHCO₃. The minor component can include products derived from the minor components such as derivatives, salts and hydrates. An example of the derived products is putrescine dihydrochloride.

Specific examples of the basal medium for pluripotent stem cells include known chemically synthesized media such as commercial Dulbecco's Modified Eagle's medium (DMEM), Minimum Essential Medium (MEM), Basal Medium Eagle (BME), RPMI1640 medium and F12 medium, media obtained by mixing any two or more of the media at an appropriate ratio such as DMEM/F12 medium (a medium obtained by mixing DMEM and F12 media at 1:1) and media obtained by adding the ascorbic acid, preferably ascorbic acid 2-phosphate, to the media.

In particular, ESF Nutrient Medium described in Miho Furue et al. In Vitro Cell Dev Biol Anim. 41, 19-28 (2005) (hereinafter also called "mESF Basal Medium") is a preferable example of an animal product-free basal medium.

A ROCK inhibitor means a substance which inhibits the activity of Rho kinase. Rho kinase is a kind of low molecular weight GTP-binding protein (low molecular weight G protein) included in the family of GTPases, which are enzymes that decompose GTP (guanosine triphosphate), and has an amino-terminal serine/threonine kinase domain, a middle coiled-coil domain and a carboxy-terminal Rho-interacting domain.

Examples of the ROCK inhibitor include (+)-(R)-trans-4-(1-aminoethyl)-N-(4-pyridyl)cyclohexanecarboxamide dihydrochloride (Y-27632), 1-(5-isoquinolinesulfonyl)-2-methylpiperazine (H-7), 1-(5-isoquinolinesulfonyl)-3-methylpiperazine (iso-H-7), N-2-(methylamino)ethyl-5-isoquinolinesulfonamide dihydrochloride (H-8), N-(2-aminoethyl)-5-isoquinolinesulfonamide dihydrochloride (H-9), N-[2-(p-bromocinnamylamino)ethyl]-5-isoquinolinesulfonamide dihydrochloride (H-89), N-(2-guanidinoethyl)-5-isoquinolinesulfonamide hydrochloride (HA-1004), 1-(5-isoquinolinesulfonyl)homopiperazine dihydrochloride (Fasudil/HA-1077), (S)-(+)-2-methyl-4-glycyl-1-(4-methylisoquinolinyl-5-sulfonyl)homopiperidine dihydrochloride (H-1152) and the like. Of these examples, (+)-(R)-trans-4-(1-aminoethyl)-N-(4-pyridyl)cyclohexanecarboxamide dihydrochloride (Y-27632) is preferable.

The concentration of the ROCK inhibitor contained in the medium of the invention is 5 µM or less, preferably 4 µM or less, more preferably 3 µM or less, still more preferably 2 µM or less, particularly preferably 1.5 µM or less. Although the lower limit of the concentration of the ROCK inhibitor is not particularly limited, the concentration is generally preferably 0.3 µM or more. When the concentration of the ROCK inhibitor exceeds 5 µM, the proportion of the naive pluripotent stem cells lowers. By adjusting the concentration at 0.3 µM or more, a decrease in the proliferation of the pluripotent stem cells can be inhibited.

It has been generally necessary to add a MEK inhibitor to the medium to maintain the naive state of pluripotent stem cells. However, because the medium of the invention contains a ROCK inhibitor at 5 µM or less, the naive state of pluripotent stem cells is maintained when pluripotent stem cells are cultured using the medium. Thus, it is not necessary that the medium contains a MEK inhibitor.

Because the medium of the invention contains a ROCK inhibitor at a concentration of 5 µM or less, apoptosis is not caused easily when pluripotent stem cells are dispersed into single cells or clusters of several to several dozen cells. Thus, the medium of the invention is preferably used also when pluripotent stem cells are cultured in suspension culture. Because pluripotent stem cells can be cultured in suspension culture in this manner, a large number of cells can be produced at low cost.

Accordingly, the medium of the invention does not have to contain feeder cells or extracellular matrix, which have been necessary for adhesive culture, when cells are cultured in suspension culture.

The medium of the invention may further contain a growth factor. The proliferation of pluripotent stem cells sometimes decreases because the ROCK inhibitor content of the medium of the invention is low. By adding a growth factor to the medium of the invention, the effect of recovering the proliferation of pluripotent stem cells is exhibited.

Examples of the growth factor include activin A, transforming growth factor-α (TGF-α), transforming growth factor-β (TGF-β), macrophage inflammatory protein-1α (MIP-1α), epidermal growth factor (EGF), fibroblast growth factor-1, 2, 3, 4, 5, 6, 7, 8 or 9 (FGF-1, 2, 3, 4, 5, 6, 7, 8 or 9), nerve growth factor (NGF), hepatocyte growth factor (HGF), leukemia inhibitory factor (LIF), protease nexin I, protease nexin II, platelet-derived growth factor (PDGF), cholinergic differentiation factor (CDF), chemokines, Notch ligands (such as Delta 1), Wnt proteins, angiopoietin-like protein 2, 3, 5 or 7 (Angpt 2, 3, 5 or 7), insulin-like growth factors (IGFs), insulin-like growth factor-binding proteins (IGFBPs), pleiotrophin, heparan sulfate and the like. Of these examples, FGF2, LIF or heparan sulfate is preferable.

One growth factor may be used in the medium of the invention, or a combination of two or more kinds may also be used. When a growth factor is used, the concentration thereof is generally preferably 0.1 to 1000 ng/mL, more preferably 1 to 20 ng/mL.

The medium of the invention may contain a GSK3 inhibitor, a BRAF inhibitor, an endogenous protein that inhibits BMP or the like. When such a compound is contained in the medium, naive pluripotent stem cells can be efficiently proliferated while the undifferentiated state and the pluripotency (pluripotent capability) of the naive pluripotent stem cells are maintained.

Examples of the GSK3 inhibitor include CHIR99021 (6-{2-[4-(2,4-dichlorophenyl)-5-(4-methyl-1H-imidazol-2-yl)-pyrimidin-2-ylamino]-ethylamino}-nicotinonitrile), CHIR98014 (2-[[2-[(5-nitro-6-aminopyridin-2-yl)amino]ethyl]amino]-4-(2,4-dichlorophenyl)-5-(1H-imidazol-1-yl)pyrimidine), AR-AO144-18, TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), 1-AzKenpaullone (9-bromo-7,12-dihydro-pyrido[3',2':2,3]azepino[4,5-b]indol-6(5H)-one), Kenpaullone (9-bromo-7,12-dihydro-indolo-[3,2-d]-[1]benzazepin-6(5H)-one) and the like, but the GSK3 inhibitor is not limited to these examples. Of these examples, CHIR99021 is preferable.

Examples of the BRAF inhibitor include GDC-0879, BAY 7304506 (regorafenib), RAF265 (CHIR-265), SB590885 and sorafenib, but the BRAF inhibitor is not limited to these examples. Of these examples, SB590885 is preferable.

The BMP inhibitor includes a compound which inhibits BMP2, BMP4, BMP6 or BMP7, which activate transcription factor SMAD1, SMAD5 or SMAD8, such as Noggin, chordin and follistatin. Of these examples, Noggin is preferable.

Examples of the MEK inhibitor include PD184352 and PD98059, which inhibit MEK1, PD0325901, U0126 and SL327, which inhibit MEK1 and MEK2, and the like, but the MEK inhibitor is not limited to these examples.

For the medium of the invention, one kind of the GSK3 inhibitor, the BRAF inhibitor, the endogenous protein that inhibits BMP or the like may be used, or a combination of two or more kinds may be used. The concentration thereof is generally preferably 0.001 to 20 µM, more preferably 0.02 to 10 µM.

The medium for culturing naive pluripotent stem cells of the invention is used for efficiently proliferating naive pluripotent stem cells while maintaining the undifferentiated state and the pluripotency (pluripotent capability) of the naive pluripotent stem cells, and a specific subject is human pluripotent stem cells.

Examples of the pluripotent stem cells include embryonic stem cells (ES cells) isolated from an early embryo; embryonic germ cells (EG cells) isolated from primordial germ cells at the prenatal stage (for example, see Proc Natl Acad Sci USA. 1998, 95:13726-31); germline stem cells (GS cells) isolated from testis just after birth (for example, see Nature. 2008, 456:344-9); stem cells derived from bone marrow such as iliac bone marrow or jaw bone marrow; mesenchymal stem cells such as adipose tissue-derived stem cells; somatic cell-derived artificial pluripotent stem cells [or induced pluripotent stem cell (iPS cells)] which are obtained by introducing genes to somatic cells such as skin cells to induce the dedifferentiation of the somatic cells of the analyte itself and which have similar pluripotency to that of ES cells.

Specific examples include H9 (WA09) human ES cells; HI (WA01) human ES cells (University of Wisconsin, WISC Bank); human ES cells such as KhES-1, KhES-2 and KhES-3 (Research Center for Stem Cell Medicine, Institute for Frontier Medical Sciences, Kyoto University and RIKEN BioResource Center) and HES3, HES4 and HES6 (Monash University, WISC Bank); iPS cells obtained by introducing Oct3/4 gene, Klf4 gene, C-Myc gene and Sox2 gene (Center for iPS Cell Research and Application, Kyoto University and RIKEN BioResource Center); Tic (line JCRB1331), Dotcom (line JCRB1327), Squeaky (line JCRB1329), Toe (line JCRB1338) and Lollipop (line JCRB1336) (Center for Child Health and Development and JCRB Cell Bank, National Institutes of Biomedical Innovation, Health and Nutrition); line UTA-1 (Tokyo University) and line UTA-1-SF-2-2 (JCRB1493) (Tokyo University and JCRB Cell Bank, National Institutes of Biomedical Innovation, Health and Nutrition); and iPS cells such as iPS cells obtained by introducing Oct3/4 gene, Klf4 gene and Sox2 gene (Nat Biotechnol 2008; 26: 101-106) (Center for iPS Cell Research and Application, Kyoto University, RIKEN BioResource Center and JCRB Cell Bank, National Institutes of Biomedical Innovation, Health and Nutrition).

### 2. Method for Culturing Pluripotent Stem Cells

Next, the method for culturing pluripotent stem cells of the invention is explained. The method for culturing pluripotent stem cells of the invention is a method characterized by culturing naive pluripotent stem cells in a medium containing a ROCK inhibitor at a concentration of 5 µM or less and thus proliferating the pluripotent stem cells while maintaining the naive state of the pluripotent stem cells (hereinafter also called "the culture method of the invention"). The ROCK inhibitor is as described above, and its preferable concentration is also as described above.

It has been generally necessary to add a MEK inhibitor to the medium to maintain the naive state of pluripotent stem cells. In the culture method of the invention, however, because the medium contains a ROCK inhibitor at 5 µM or less, the naive state of pluripotent stem cells is maintained when pluripotent stem cells are cultured using the medium. Thus, it is not necessary that the medium contains a MEK inhibitor in the culture method of the invention.

The culture method of the invention is preferably conducted in suspension culture. When cells are cultured in suspension culture, a large number of cells can be produced at low cost. Thus, in the culture method of the invention, it is not necessary to culture cells using feeder cells or extracellular matrix, which have been necessary for adhesive culture, when the cells are cultured in suspension culture.

In the culture method of the invention, a growth factor may be used to increase the proliferation efficiency of the pluripotent stem cells. The kind of the growth factor used in the culture method of the invention and the amount added to the medium are as described above.

In the culture method of the invention, a GSK3 inhibitor, a BRAF inhibitor, an endogenous protein that inhibits BMP or the like may be used. The kind of the GSK3 inhibitor, the BRAF inhibitor, the endogenous protein that inhibits BMP or the like and the amount added to the medium are as described above.

The cultivation in the culture method of the invention is preferably conducted at generally 33 to 40°C, preferably 34 to 39°C, more preferably 37°C, in the presence of carbon dioxide at generally 1 to 20%, preferably 3 to 15%, more preferably 8 to 10%, in high humidity of generally 75% or higher, preferably 85% or higher, more preferably 95%, particularly preferably 100%.

The pluripotent stem cells which can be used are as described above.

Examples of the method for determining whether the cells cultured by the culture method of the invention maintain the undifferentiated state and the naive state include a method for determining through the detection of expression of an undifferentiation marker and/or the non-detection of expression of a differentiation marker using an antibody to a marker (protein), a method for determining through the detection of expression of a marker (gene), a method through the observation of the morphological features of the cells and a method for determining whether the cells can exhibit the ability to differentiate into specific cells through stimulation by a specific differentiation-inducing factor.

In the methods for determining using a marker, it can be determined that the pluripotent stem cells cultured by the culture method of the invention maintain the undifferentiated state when an undifferentiation marker such as NANOG, OCT3/4, SSEA-3, SSEA-4, TRA-2-54, TRA-1-60, TRA-1-80, CD90 or alkaline phosphatase is expressed. Moreover, when a marker such as STELLA is expressed, it can be determined that the pluripotent stem cells cultured by the culture method of the invention are the naive type.

The expression of the undifferentiation and/or differentiation marker can be examined at the protein level for example using a specific antibody to the marker by flow cytometry, immunostaining, ELISA or the like. The expression of the marker can be examined at the gene level for example by RT-PCR using a specific primer pair for the marker gene, northern blotting using a specific probe, microarray analysis or the like.

An example of the method for examining whether or not a marker is expressed by flow cytometry is the following method. The cells cultured by the method of the invention are suspended in 1 ml of 10% goat serum for 30 minutes and then centrifuged. Next, the cells are incubated for 30 hours with a mouse antibody to the target marker protein. Then, the cultured cells are washed three times with PBS containing 1% goat serum, reacted with a fluorescently labeled goat anti-mouse IgG antibody for 30 minutes and washed three times with PBS containing 1% goat serum. The cells which are suspended again can be examined using an appropriate flow cytometry device.

An example of the method for examining whether or not a marker is expressed by immunostaining is the following method. The cells cultured by the method of the invention are fixed with 4% paraformaldehyde (PFA) in PBS and then washed more than once with PBS. Then, the permeability of the cultured cells is increased using Triton X or the like. After blocking with 10% goat serum, the cells are immunostained using a mouse antibody to the target marker protein, reacted with fluorescently labeled goat anti-mouse IgG and observed with a fluorescence microscope for the determination.

An example of the method for examining whether or not alkaline phosphatase is expressed is the following method. The cells cultured by the method of the invention are fixed for five minutes with 4.5 mM citric acid, 2.25 mM sodium citrate, 3 mM sodium chloride, 65% methanol and 4% paraformaldehyde and washed, and then alkaline phosphatase can be visualized using an appropriate kit such as FastRed substrate kit (manufactured by Sigma, Inc.), which is so-called alkaline phosphatase staining.

### Examples

Next, the invention is explained in further detail referring to Examples, but the invention is not limited by these Examples.

### [Example 1]

### Influence on Proliferation of iPS Cells in the Presence of ROCK Inhibitor

In the following Examples, a medium obtained by mixing the following components in the amounts shown in Table 1 which had been sterilized with a 20 µm filter into mESF Basal Medium (manufactured by Wako Pure Chemical Industries, Ltd., 136-17805) was used as hESF-FX medium.

rHSA-Oleic acid was prepared by adding 235 µL of 200 mg/mL oleic acid (manufactured by Sigma-Aldrich, Inc., O1383-5G) to 50 mL of 100 mg/mL recombinant human serum albumin (rHSA, manufactured by Millipore Corporation, 9501-20) and mixing the mixture overnight.

**[Table 1]**

| Components | Final Concentration |
|---|---|
| 2-Mercaptoethanol | 10 µM |
| Sodium selenite | 20 µM |
| Ethanolamine | 10 µM |
| Recombinant human serum albumin (rHSA)-Oleic acid | 1 mg/mL |
| Human insulin, recombinant (manufactured by Sigma-Aldrich, Inc., I9278-5ML) | 10 µg/mL |
| Apo-transferrin, human (manufactured by Sigma-Aldrich, Inc., T-2252-100MG) | 5 µg/mL |
| Ascorbic acid 2-phosphate (manufactured by Wako Pure Chemical Industries, Ltd., 013-19641) | 0.1 mg/mL |
| Human fibroblast growth factor-2 (FGF-2), recombinant (manufactured by R&D Systems, Inc., 233-FB-025/CF) | 5 ng/mL |
| Human activin A, recombinant (manufactured by R&D Systems, Inc., 338-AC-010/CF) | 3 ng/mL |

As the iPS cells, Tic-FX cells [a cell line obtained by conditioning iPS cells, line Tic (JCRB1331) to a feeder-free xeno-free culture (FX)] were used.

A ROCK inhibitor (Y-27632) was dissolved in the hESF-FX medium at 1 µM, 3 µM or 10 µM. The media each in an amount of 3 mL were put into an ultra-low attachment 6-well plate (manufactured by Corning Incorporated), and the Tic-FX cells were cultured in the presence of 10% CO₂ at 37°C (suspension culture).

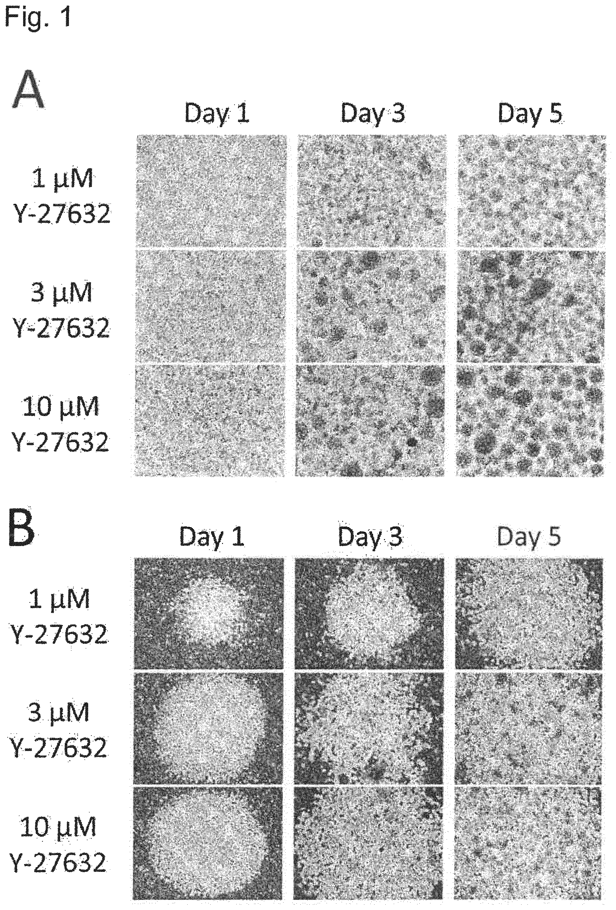

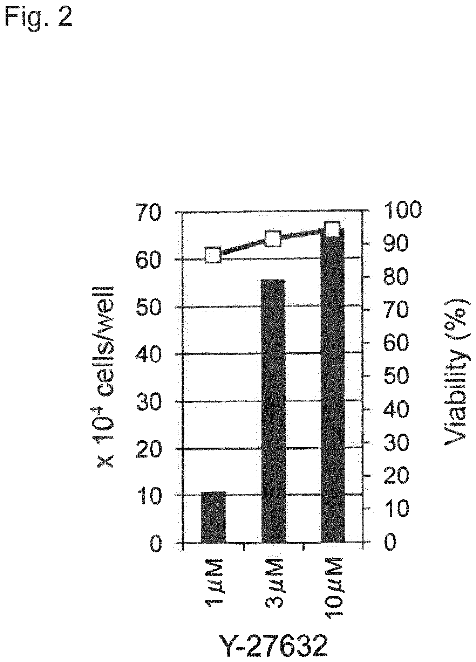

The plate was observed after one day, three days and five days of cultivation. The results are shown in Fig. 1A and Fig. 1B. Fig. 1A shows 4× micrographs, and Fig. 1B shows 2× micrographs. The numbers of the cells per well and the viability of the cells were measured after five days of cultivation. The results are shown in Fig. 2.

As shown in Fig. 1A and Fig. 1B, the number of the cells increased as the concentration of the ROCK inhibitor was higher. Also, as shown in Fig. 2, the number of the cells per well and the viability of the cells both increased as the concentration of the ROCK inhibitor was higher.

### [Example 2]

### Expression of Undifferentiation Marker Genes and Naive Type-Specific Marker Gene

The expression of marker genes was examined by real-time PCR. Real-time PCR was conducted using a real-time PCR system (7300 manufactured by Applied Biosystems) by the following reaction procedures using the following primers.

### (Reaction Procedures)

95°C 30 seconds → [(95°C 5 seconds → 60°C 31 seconds) × 40 cycles] → Melting Cycle

### (Primers)

NANOG forward primer tgaacctcagctacaaacag (SEQ ID NO: 1)
NANOG reverse primer tggtggtaggaagagtaaag (SEQ ID NO: 2)
OCT3/4 forward primer gacagggggaggggaggagctagg (SEQ ID NO: 3)
OCT3/4 reverse primer cttccctccaaccagttgccccaaa (SEQ ID NO: 4)
STELLA forward primer gttactgggcggagttcgta (SEQ ID NO: 5)
STELLA reverse primer tgaagtggcttggtgtcttg (SEQ ID NO: 6)
NCAM forward primer ggcatttacaagtgtgtggttac (SEQ ID NO: 7)
NCAM reverse primer ttggcgcattcttgaacatga (SEQ ID NO: 8)
GAPDH forward primer caaagttgtcatggatgacc (SEQ ID NO: 9)
GAPDH reverse primer ccatggagaaggctgggg (SEQ ID NO: 10)

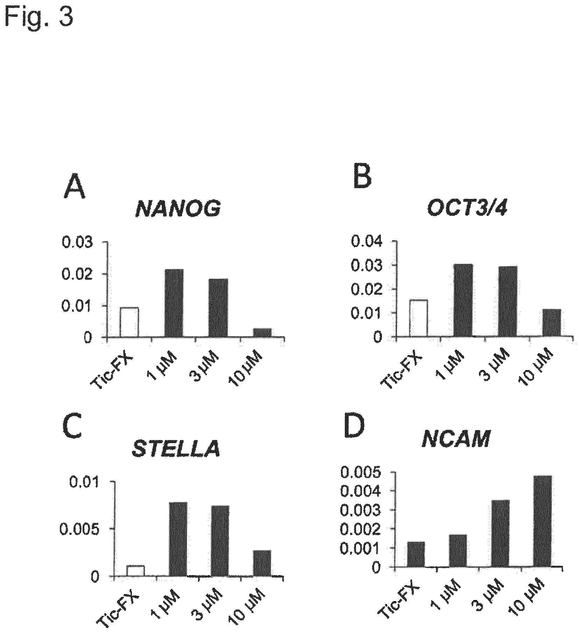

Using the cells cultured in Example 1, the expression levels of NANOG and OCT3/4, which are undifferentiation marker genes, STELLA, which is a naive type-specific marker gene, and NCAM, which is a neuronal differentiation-specific marker gene, in the cells after five days of cultivation were examined by the real-time PCR. The results are shown in Fig. 3A to Fig. 3D.

In Fig. 3A to Fig. 3D, the vertical axes each indicate the ratio of the expression level of the target gene of the sample to that of GAPDH (glyceraldehyde-3-phosphate dehydrogenase), which was used as the internal standard. Specifically, the values in the graphs each mean the relative value (unit is fold) of the DNA amount before the amplification determined from the difference between the Cp value of GAPDH and the Cp value of the target gene.

Here, the internal standard is a gene whose expression level is always constant independently of the state of the cells. The Cp value is the number of cycles which are necessary to amplify DNA to the amount that can be detected with a measurement device when the DNA is amplified by PCR reaction (theoretically, the DNA amount doubles in one cycle).

As shown in Fig. 3A and Fig. 3B, the expression levels of NANOG and OCT3/4, which are undifferentiation marker genes, were higher than those of the control (Tic-FX in Fig. 3A and Fig. 3B) when the concentration of the ROCK inhibitor was 1 µM and 3 µM, while the expression levels of both genes were lower than those of the control when the ROCK inhibitor was used at a concentration of 10 µM.

As shown in Fig. 3C, the expression level of naive type-specific STELLA gene was slightly higher than that of the control when the concentration of the ROCK inhibitor was 10 µM, while the expression levels were higher than that of the control when the concentration of the ROCK gene was 1 µM and 3 µM.

From the results, it was found that naive pluripotent stem cells can be efficiently proliferated while maintaining the undifferentiated state when a ROCK inhibitor is used at a lower concentration (1 µM and 3 µM) than the conventionally used concentration of 10 µM.

Moreover, as shown in Fig. 3D, the expression level of NCAM, which is a middle lobe differentiation-specific marker gene, was almost the same as that of the control when the concentration of the ROCK inhibitor was 1 µM, and the expression levels were higher than that of the control when the concentration was 3 µM and 10 µM.

### [Example 3]

### Proliferation of iPS Cells in the Presence of Growth Factors

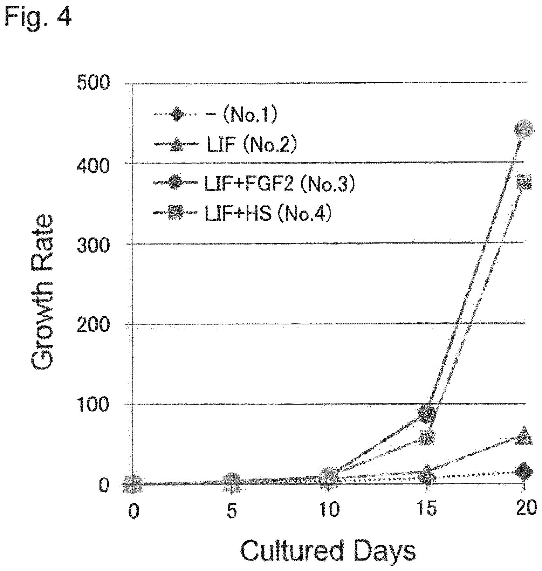

Media were produced by adding 1 µM Y-27632 and the components shown in Table 2 to the basal medium, and the Tic-FX cells were proliferated in suspension culture in an ultra-low attachment 6-well plate (manufactured by Corning Incorporated) in the presence of 10% CO₂ at 37°C. The cells were subcultured five days, 10 days and 15 days after seeding and cultured until day 20. The cells were subcultured by treating cell clusters using Accutase (manufactured by Millipore Corporation, SCR005) at 37°C for 10 minutes. The cells were counted five days, 10 days, 15 days and 20 days after seeding. The results are shown in Fig. 4.

**[Table 2]**

| No. | Recombinant human LIF (leukemia inhibitory factor) | Recombinant human FGF2 (fibroblast growth factor 2) | Sodium heparan sulfate |
|---|---|---|---|
| 1 | - | 5 ng/mL | - |
| 2 | 20 ng/mL | 5 ng/mL | - |
| 3 | 20 ng/mL | 40 ng/mL | - |
| 4 | 20 ng/mL | 5 ng/mL | 100 ng/mL |

As shown in Fig. 4, the growth rates in the medium containing 5 ng/mL recombinant human FGF2 (No. 1) and the medium containing 5 ng/mL recombinant human FGF2 and 20 ng/mL recombinant human LIF (No. 2) were low in the presence of 1 µM Y-27632. On the other hand, the growth rates increased in the medium containing 40 ng/mL FGF2 and 20 ng/mL recombinant human LIF (No. 3) and the medium containing recombinant human LIF, recombinant human FGF2 and sodium heparan sulfate at 20 ng/mL, 5 ng/mL and 100 ng/mL, respectively (No. 4).

From the results, it was found that it is preferable to add a growth factor such as recombinant human LIF, recombinant human FGF2 and sodium heparan sulfate to the medium together with a ROCK inhibitor at a low concentration in order to efficiently culture suspension cells.

### [Example 4]

### Expression of Undifferentiation Marker Genes and Naive Type-Specific Marker Genes

Using the cells cultured in Example 3 (the cells of Nos. 3 and 4 in Table 2), the expression of marker genes was examined by real-time PCR in the same manner as in Example 2. The nucleotide sequences of the primers used for examining the expression of TFCP2L1 gene are shown below.

TFCP2L1 forward primer tgtgaggccaaagatgaccattta (SEQ ID NO: 11)
TFCP2L1 reverse primer caatcagctccaaggtggtcag (SEQ ID NO: 12)

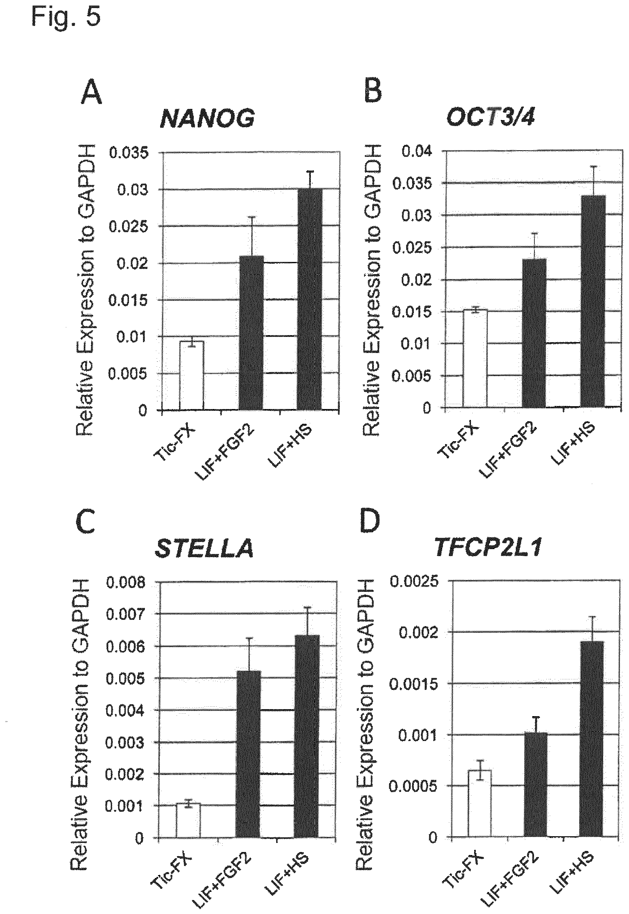

The results are shown in Fig. 5A to Fig. 5D. In this regard, a medium to which none of recombinant human LIF, recombinant human FGF2 and sodium heparan sulfate was added was used as the control (indicated by Tic-FX in Fig. 5A to Fig. 5D).

As shown in Fig. 5A and Fig. 5B, it was found that, even in the presence of Y-27632, which is a ROCK inhibitor, at a low concentration, the expression levels of NANOG and OCT3/4, which are pluripotency markers (undifferentiation marker genes), increased when growth factors were added to the medium. Moreover, as shown in Fig. 5C and Fig. 5D, it was found that naive type-specific STELLA gene and TFCP2L1 gene were expressed in the both cases.

### [Example 5]

### Culturing of iPS Cells in the Presence of Low Molecular Weight Compounds

Media were produced by adding 1 µM of Y-27632, 20 ng/mL of LIF and 40 ng/mL of FGF2 to the basal medium, and media were produced by adding the following inhibitors to the media at concentrations of 1 µM (PD0325901), 0.3 µM (CHIR99021), 0.5 µM (SB590885), 1 µM (WH-4-023), 10 µM (SP600125), 10 µM (SB203580), 5 µM (Go6983), 5 µM (GF109203X), 2 µM (Dorsomorphin) or 400 ng/mL (Noggin) each.

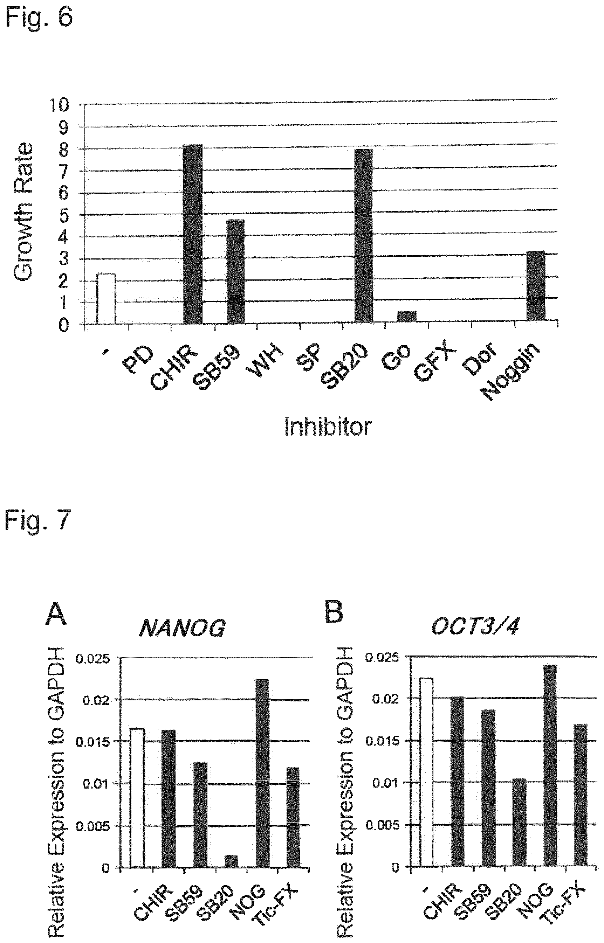

The Tic-FX cells were proliferated in suspension culture in ultra-low attachment 6-well plates (manufactured by Corning Incorporated) containing the media in the presence of 10% CO₂ at 37°C. The cells were cultured for five days, and the cells were counted. The results are shown in Fig. 6. In Fig. 6, "-" indicates the results using the medium to which no inhibitor was added.

### (Inhibitors Added to Media)

PD0325901: N-[(2R)-2,3-dihydroxypropoxy]-3,4-difluoro-2-[(2-fluoro-4-iodophenyl)amino]-benzamide, manufactured by TOCRIS, 4192
CHIR99021: 6-{2-[4-(2,4-dichloro-phenyl)-5-(4-methyl-1H-imidazol-2-yl)-pyrimidin-2-ylamino]-ethylamino}-nicotinonitrile, manufactured by TOCRIS, 4423
SB590885: 5-(2-(4-(2-dimethylaminoethoxy)phenyl)-5-(4-pyridinyl)-1H-imidazol-4-yl)-2,3-dihydro-1H-indoen-1-one oxime, manufactured by R&D Systems, Inc., 2650/10
WH-4-023: 2,6-dimethylphenyl N-(2,4-dimethoxyphenyl)-N-[2-[[4-(4-methyl-1-piperazinyl)phenyl]amino]-4-pyrimidinyl] carbamate, manufactured by MedChem Express, HY-12299
SP600125: 1,9-pyrazoloanthrone, manufactured by Sigma-Aldrich, Inc., S5567-10MG
SB203580: 4-(4-fluorophenyl)-2-(4-methylsulfinylphenyl)-5-(4-pyridyl) 1H-imidazole, manufactured by Wako Pure Chemical Industries, Ltd., 193-15611
Go6983: 3-[1-[3-(dimethylamino)propyl]-5-methoxy-1H-indol-3-yl]-4-(1H-indol-3-yl)-1H-pyrrole-2,5-dione, manufactured by Cayman Chemical, 13311
GF109203X: 3-(N-[dimethylamino]propyl-3-indolyl)-4-(3-indolyl)maleimide, 3-[1-[3-(dimethylamino)propyl] 1H-indol-3-yl]-4-(1H-indol-3-yl) 1H-pyrrole-2,5-dione, manufactured by TOCRIS, 741
Dorsomorphin: 6-[4-(2-piperidin-1-ylethoxy)phenyl]-3-pyridin-4-ylpyrazolo[1,5-a]pyrimidine, manufactured by Sigma-Aldrich, Inc., P5499-5MG
Noggin, Recombinant Human: manufactured by R&D Systems, Inc., 6057-NG-025/CF)

As shown in Fig. 6, the growth rates of the cells were high in the media to which CHIR99021, SB590885 or Noggin was added.

Next, the expression of pluripotency markers (NANOG and OCT3/4) in the cells proliferated in media to which the inhibitors resulted in high cell growth rates were added was examined by real-time PCR in the same manner as in Example 2. The results are shown in Fig. 7A and Fig. 7B. Cells cultured in the basal medium were used as the control (indicated by Tic-FX in Fig. 7A and Fig. 7B).

As it is obvious from Fig. 7A and Fig. 7B, it was found that the expression levels of NANOG and OCT3/4 increased in the media to which CHIR99021, SB590885 or Noggin was added.

From the results, it was found that the productivity can be further improved while maintaining the undifferentiated state of cells by further adding CHIR99021, SB590885 or Noggin in addition to the medium of the invention.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2016-068718) filed on March 30, 2016 and the entire contents of which are incorporated herein by reference. Further, all references cited herein are incorporated herein by reference in their entirety.

## Claims

1. A medium for culturing naive pluripotent stem cells containing a ROCK inhibitor at a concentration of 5 µM or less.

2. The medium for culturing naive pluripotent stem cells according to claim 1, wherein the ROCK inhibitor is Y-27632.

3. The medium for culturing naive pluripotent stem cells according to claim 1 or 2 which does not contain a MEK inhibitor.

4. The medium for culturing naive pluripotent stem cells according to any one of claims 1 to 3 which does not contain feeder cells and extracellular matrix.

5. The medium for culturing naive pluripotent stem cells according to any one of claims 1 to 4 which further contains a growth factor.

6. The medium for culturing naive pluripotent stem cells according to any one of claims 1 to 5 which is used for suspension culture.

7. The medium for culturing naive pluripotent stem cells according to any one of claims 1 to 6, wherein the naive pluripotent stem cells are human iPS cells.

8. A method for culturing pluripotent stem cells **characterized by** culturing naive pluripotent stem cells in a medium containing a ROCK inhibitor at a concentration of 5 µM or less and thus proliferating the pluripotent stem cells while maintaining the naive state of the pluripotent stem cells.

9. The method for culturing pluripotent stem cells according to claim 8, wherein the ROCK inhibitor is Y-27632.

10. The method for culturing pluripotent stem cells according to claim 8 or 9, wherein the medium does not contain a MEK inhibitor.

11. The method for culturing pluripotent stem cells according to any one of claims 8 to 10, wherein the medium does not contain feeder cells and extracellular matrix.

12. The method for culturing pluripotent stem cells according to any one of claims 8 to 11 which is conducted in suspension culture.

13. The method for culturing pluripotent stem cells according to any one of claims 8 to 12, wherein the naive pluripotent stem cells are human iPS cells.


SEQUENCE LISTING

<110>  National Institutes of Biomedical Innovation, Health and
       Nutrition
       KYOWA HAKKO BIO CO., LTD.

<120>  The medium for culturing naive type pluripotent stem cells and
       the method of culturing pluripotent stem cells

<130>  W522473

<150>  JP 2016-068718
<151>  2016-03-30

<160>  12

<170>  PatentIn version 3.5

<210>  1
<211>  20
<212>  DNA
<213>  Artificial Sequence

<220>
<223>  Artificially synthesized oligonucleotide

<400>  1
tgaacctcag ctacaaacag                                                  20


<210>  2
<211>  20
<212>  DNA
<213>  Artificial Sequence

<220>
<223>  Artificially synthesized oligonucleotide

<400>  2
tggtggtagg aagagtaaag                                                  20


<210>  3
<211>  24
<212>  DNA
<213>  Artificial Sequence

<220>
<223>  Artificially synthesized oligonucleotide

<400>  3
gacaggggga ggggaggagc tagg                                             24


<210>  4
<211>  25
<212>  DNA
<213>  Artificial Sequence

<220>
<223>  Artificially synthesized oligonucleotide

<400>  4

cttccctcca accagttgcc ccaaa 25

<210> 5
<211> 20
<212> DNA
<213> Artificial Sequence

<220>
<223> Artificially synthesized oligonucleotide

<400> 5
gttactgggc ggagttcgta 20

<210> 6
<211> 20
<212> DNA
<213> Artificial Sequence

<220>
<223> Artificially synthesized oligonucleotide

<400> 6
tgaagtggct tggtgtcttg 20

<210> 7
<211> 23
<212> DNA
<213> Artificial Sequence

<220>
<223> Artificially synthesized oligonucleotide

<400> 7
ggcatttaca agtgtgtggt tac 23

<210> 8
<211> 21
<212> DNA
<213> Artificial Sequence

<220>
<223> Artificially synthesized oligonucleotide

<400> 8
ttggcgcatt cttgaacatg a 21

<210> 9
<211> 20
<212> DNA
<213> Artificial Sequence

<220>
<223> Artificially synthesized oligonucleotide

<400> 9
caaagttgtc atggatgacc 20

<210>  10
<211>  18
<212>  DNA
<213>  Artificial Sequence

<220>
<223>  Artificially synthesized oligonucleotide

<400>  10
ccatggagaa ggctgggg                                                    18


<210>  11
<211>  24
<212>  DNA
<213>  Artificial Sequence

<220>
<223>  Artificially synthesized oligonucleotide

<400>  11
tgtgaggcca aagatgacca ttta                                             24


<210>  12
<211>  22
<212>  DNA
<213>  Artificial Sequence

<220>
<223>  Artificially synthesized oligonucleotide

<400>  12
caatcagctc caaggtggtc ag                                               22